# FASCICULE DE BREVET EUROPEEN

(11) **EP 2 731 622 B1**
(45) Date de publication et mention de la délivrance du brevet: **11.10.2017**
(21) Numéro de dépôt: 12744086.5
(22) Date de dépôt: 12.07.2012
(51) Int. Cl.: A61K 39/39

(54) **COMPOSITION VACCINALE AVEC DES NANOPARTICULES D'HYDROXYDE D'ALUMINIUM**
IMPSTOFFZUSAMMENSETZUNG MIT ALUMINIUMHYDROXID-NANOPARTIKELN
VACCINE COMPOSITION WITH ALUMINIUM HYDROXIDE NANOPARTICLES

(30) Priorité: 13.07.2011 FR 1156398
(43) Date de publication de la demande: 21.05.2014
(73) Titulaire: Sanofi Pasteur, 69367 Lyon Cedex 07 (FR)
(72) Inventeur: MATHIEU, Yannick, 68000 Colmar (FR); LEBEAU, Bénédicte, 68700 Wattwiller (FR); VALTCHEV, Valentin, 14610 Basly (FR); PATARIN, Joël, 68720 Flaxlanden (FR); GARINOT, Marie, 69003 Lyon (FR); HAENSLER, Jean, 69290 Grezieu La Varenne (FR); SAUZEAT, Elisabeth, 69210 Lentilly (FR)
(74) Mandataire: Kerneis, Daniéle
(86) Numéro de dépôt international: PCT/FR2012/051648
(87) Numéro de publication internationale: WO 2013/007956

(56) Documents cités:
- EP-A2- 1 126 876
- WO-A1-94/15636
- WO-A1-2007/052058
- WO-A2-2008/109852
- US-A- 4 269 821
- US-A1- 2005 158 334
- STIENEKER F ET AL: "Comparison of 24 different adjuvants for inactivated HIV-2 split whole virus as antigen in mice. Induction of titres of binding antibodies and toxicity of the formulations", VACCINE, ELSEVIER LTD, GB, vol. 13, no. 1, 1 janvier 1995 (1995-01-01), pages 45-53, XP004057700, ISSN: 0264-410X, DOI: 10.1016/0264-410X(95)80010-B
- YANNICK MATHIEU ET AL: "Control of the Morphology and Particle Size of Boehmite Nanoparticles Synthesized under Hydrothermal Conditions", LANGMUIR, vol. 23, no. 18, 1 août 2007 (2007-08-01), pages 9435-9442, XP055024971, ISSN: 0743-7463, DOI: 10.1021/la700233q cité dans la demande
- KWOK PAN YAU ET AL: "Aluminum hydroxide adjuvant produced under constant reactant concentration", JOURNAL OF PHARMACEUTICAL SCIENCES, vol. 95, no. 8, 1 août 2006 (2006-08-01), pages 1822-1833, XP055024975, ISSN: 0022-3549, DOI: 10.1002/jps.20692
- COIAI SERENA ET AL: "Organophilic Boehmite nanoparticles by ATRP methacrylates polymerization: synthesis, characterization and dispersion in polypropylene", JOURNAL OF NANOSCIENCE AND NANOTECHNOLOGY, AMERICAN SCIENLIFLC PUBLISHERS, US, vol. 8, no. 4, 1 avril 2008 (2008-04-01), pages 1803-1811, XP009158586, ISSN: 1533-4880
- ALPHONSE ET AL: "Surface and porosity of nanocrystalline boehmite xerogels", JOURNAL OF COLLOID AND INTERFACE SCIENCE, ACADEMIC PRESS, NEW YORK, NY, US, vol. 290, no. 1, 1 octobre 2005 (2005-10-01), pages 208-219, XP005037385, ISSN: 0021-9797

## Description

La présente invention est relative au domaine des vaccins et plus particulièrement aux compositions vaccinales comprenant au moins un adjuvant. En particulier, l'invention concerne une composition vaccinale comprenant des nanoparticules filtrables stérilement comprenant de la pseudo-boehmite et du polyacrylate (PAA).

Il est connu depuis très longtemps dans l'art antérieur que l'aluminium est intéressant pour adjuver les vaccins. De nombreux vaccins commercialisés en contiennent, soit sous forme d'hydroxyde, soit sous forme de phosphates ; ces dénominations ne reflétant d'ailleurs pas exactement la composition chimique des produits correspondants : les hydroxydes d'aluminium sont plutôt des oxyhydroxydes, et les phosphates d'aluminium sont rarement des phosphates purs, mais contiennent très souvent d'autres ions, notamment des sulfates, ainsi que des hydroxydes. Si ces adjuvants à base d'aluminium, encore appelés gels d'aluminium ont montré tout leur intérêt pour augmenter la réponse immunitaire induite par un antigène, ils présentent cependant quelques inconvénients. D'un point de vue industriel, les suspensions traditionnelles d'hydroxyde ou de phosphate d'aluminium ne permettent pas, à cause de la taille trop grande des particules, de procéder en fin de production, à une stérilisation par filtration et imposent donc de recourir à un procédé de production se déroulant dans des conditions aseptiques. D'autre part, dans le cas de certains modes d'administration, notamment la voie intradermique, il a été reproché à l'aluminium de conduire à un effet tatouage au site d'administration. WO2007052058 divulgue une composition vaccinale comprenant un adjuvant pouvant comprendre des sels d'aluminium ou des particules comprenant un polymère tel que du polycyanoacrylate. Il est donc souhaitable de pouvoir disposer de compositions vaccinales comprenant de l'aluminium afin de bénéficier de ses pouvoirs adjuvant, tout en évitant ses inconvénients. A cette fin, la présente invention propose une composition vaccinale comprenant au moins un antigène et un adjuvant, caractérisée en ce que l'adjuvant comprend des nanoparticules filtrables stérilement comprenant de la pseudo-boehmite et du polyacrylate.

La présente invention a également pour objet l'utilisation de nanoparticules filtrables stérilement comprenant de la pseudo-boehmite et du polyacrylate pour la préparation d'une composition vaccinale comprenant au moins un antigène.

En particulier, l'invention a pour l'utilisation de telles nanoparticules qui permettent d'augmenter la réponse immunitaire induite lors de l'administration de ladite composition vaccinale.

La présente invention a encore pour objet un procédé de préparation d'une composition vaccinale comprenant au moins un antigène et un adjuvant, selon lequel :
∘ on prépare des nanoparticules filtrables stérilement comprenant de la pseudo-boehmite et du polyacrylate,
∘ on filtre lesdites nanoparticules au moyen d'un filtre stérilisant,
∘ on ajoute auxdites nanoparticules au moins un antigène vaccinal,
∘ et, de façon optionnelle, on procède si nécessaire à une filtration supplémentaire.

Grâce à l'objet de l'invention, il est possible d'avoir un adjuvant vaccinal induisant moins de réaction de réactogénicité que les suspensions d'aluminium utilisées classiquement, notamment après administration par voie intradermique (ID) et permettant d'effectuer en fin de procédé de fabrication, une stérilisation par filtration.
D'autres avantages de l'invention apparaitront au cours de la description qui suit.

Aux termes de la présente invention, les nanoparticules sont des particules dont la taille permet leur passage à travers un filtre stérilisant, dont les pores ont un diamètre de 220 nm. Les particules ont donc une taille inférieure à 300 nm, car par déformation, de telles particules peuvent passer dans des pores de plus petite taille. Mais de façon préférée, les particules ont une taille inférieure à 220 nm, et même inférieure à 200 nm. De telles particules forment des suspensions colloïdales qui sont transparentes. Avantageusement, on utilise des suspensions dont la majorité des particules ont entre 30 et 200 nm de diamètre, ce qui permet de les filtrer sans trop de perte de matière, avec un filtre stérilisant dont le seuil de coupure est 220 nm. Le diamètre des particules est un diamètre hydrodynamique qui peut être mesuré par différentes techniques ; par exemple on peut utiliser la diffusion quasi-élastique de la lumière. Cette technique permet de mesurer la taille des particules dans une suspension colloïdale pour des rayons allant de 1 nanomètre à quelques micromètres. Les particules en suspension dans un liquide sont soumises au mouvement brownien (agitation thermique et chocs entre molécules du liquide et particules solides). Le principe d'une mesure en diffusion de la lumière consiste à bombarder les particules dans la suspension colloïdale par un rayonnement cohérent et monochromatique type laser puis d'enregistrer les fluctuations de l'intensité lumineuse diffusée par ces particules à l'aide d'une photodiode à avalanche (dispositif permettant de compter le nombre de photons).
Ces expériences ont habituellement lieu dans des milieux très dilués transparents à l'oeil. Dans le cas de la présente invention, le diamètre des nanoparticules a été déterminé après dilution (généralement 1/10) dans de l'eau déminéralisée. Le modèle de l'appareil utilisé était un Malvern ZetaSizer Nano ZS.
Selon l'invention, les nanoparticules sont des particules constituées essentiellement de pseudo-boehmite avec à leur surface du polyacrylate permettant d'éviter l'agrégation des nanoparticules après lavage des nanoparticules par dialyse. Selon un mode particulier de l'invention, le polyacrylate est lié à la pseudo-boehmite par interactions de type électrostatique. Selon un mode de l'invention, la quantité de polyacrylate représente une masse inférieure à 10% de la masse des nanoparticules, plus particulièrement une masse inférieure à 9%, à 8%, à 7%, à 6%, à 5%, à 4%, à 3%, à 2%, à 1%. Selon un mode de l'invention, la quantité de polyacrylate représente une masse au moins égale à 1% de la masse des nanoparticules, plus particulièrement une masse supérieure à 2%, à 3%, à 4%, à 5%, à 6%, à 7%, à 8%, à 9%.
La qualification du constituant essentiel des nanoparticules de l'invention, en pseudo-boehmite peut être effectuée par analyse de diffraction aux rayons X, qui conduit à un diffractogramme de rayons X caractéristique de pseudo-boehmite avec les principales raies (020), (021), (130), (150), (151), et (132) observées à 14, 28.1, 32.4, 49.3, 55.5 et 65° 28 (λ=0.15406 nm).

On utilise du polyacrylate car les polymères utilisés doivent être biocompatibles, c'est-à-dire ne pas présenter de toxicité pour l'organisme auquel on les administre. En outre, le polymère utilisé doit être stable aux températures mises en oeuvre lors de la synthèse des nanoparticules. Le polyacrylate utilisé peut être de différents poids moléculaire ; il peut notamment s'agir de polyacrylate de sodium ou de tout autre sel convenant pour un usage pharmaceutique. On a obtenu de bons résultats avec le polyacrylate de sodium PAA 2100 fourni par la Société Fluka, ainsi qu'avec le PAA-60 000 de Polysciences.

Les particules sont préparées selon une méthode inspirée de la méthode décrite par S. Musić et coll. dans Materials Chemistry and Physics, 1999, 59, 12-19 ayant pour titre « *Chemical and microstructural properties of Al-oxide phases obtained from AlCl₃ solutions in alkaline medium* ». Selon S. Musić et coll., on augmente le pH d'une solution aqueuse de sel d'aluminium par ajout d'une base (NaOH) jusqu'à une valeur légèrement supérieure à 11 en un laps de temps de 5 min (contrôle à l'aide d'un pH mètre). Après quoi, le mélange est agité vigoureusement pendant 10 min puis transféré dans un autoclave et chauffé à 160°C pendant 24h. Le produit (solution blanchâtre et homogène) est récupéré par filtration sur Büchner, lavé puis séché une nuit dans une étuve à 65°C. Dans le cas de synthèses selon la présente invention, et ainsi que cela a été décrit par Y. Mathieu et coll. dans « Control of the morphology and particle size of boehmite nanoparticules synthesized under hydrothermal conditions » dans Langmuir 2007, 23, 9435-9442, la synthèse est effectuée en présence de polyacrylate de sodium que l'on ajoute au départ dans la solution aqueuse de sels d'aluminium. La présence de polymère va empêcher l'agglomération des particules formées. Les quantités relatives de polyacrylate et de sel d'aluminium sont sélectionnées afin de permettre de solubiliser le polyacrylate dans la solution de sel d'aluminium et d'obtenir des nanoparticules filtrables convenant comme adjuvant vaccinal. On a ainsi remarqué qu'avec le PAA 2100, il était avantageux de respecter un rapport molaire polyacrylate de sodium sur Aluminium compris entre 0,43 et 0,57.

Avant d'ajouter la base, le mélange polyacrylate de sodium et sel d'aluminium est soumis à une phase de murissement durant laquelle il est agité. Cette phase de murissement est avantageusement effectuée à température ambiante pendant environ 24 heures. D'un point de vue industriel, il est très intéressant de pouvoir travailler à température ambiante, sur une durée courte qui peut être prolongée si nécessaire pendant les week-ends par exemple.

Les sels d'aluminium permettant de produire des nanoparticules peuvent être de nature diverse ; il peut notamment s'agir de chlorure d'aluminium AlCl₃, de nitrate d'aluminium Al(NO₃)₃ ou encore de sulfate d'aluminium Al₂(SO₄)₃ ; de préférence, on choisit le chlorure d'aluminium AlCl₃.

Les bases que l'on peut utiliser pour augmenter le pH du mélange sont diverses et peuvent notamment être choisies parmi NaOH, KOH, ou NH₄OH. De façon avantageuse, on effectue la synthèse des nanoparticules en utilisant de la soude NaOH.

Selon l'invention, la soude est utilisée pour augmenter le pH jusqu'à une valeur d'environ 10-11 lors d'une opération durant entre 5 et 10 minutes. Durant cette phase d'augmentation de pH, les différentes espèces aluminiques en présence connaissent des modifications, telles que des dissociations et des réarrangements afin d'arriver à la formation d'une phase plus ou moins cristalline.

Selon l'invention, le mélange est ensuite soumis à un traitement hydrothermal pendant une durée pouvant aller de 1 à 17 heures ; de façon avantageuse au regard de la taille des particules obtenues et de leur filtrabilité, on choisit une durée de 3 heures. Ce traitement est effectué soit en mode statique, soit sous agitation à une température comprise entre 90°C et 200°C, plus particulièrement à 160°C.

Selon l'invention, les nanoparticules obtenues sont filtrables stérilement ce qui signifie qu'elles ont une taille leur permettant de traverser un filtre stérilisant dont la taille des pores est de 220 nm ; les pertes observées lors d'une telle filtration sont compatibles avec des contraintes industrielles. En effet, des essais effectués sur des filtres PTFE (Polytetrafluoroéthylène) 0.22µm ont montré que l'on avait moins de 5% de perte en polymère et en aluminium, cette valeur de 5% pouvant correspondre à la marge d'erreur de la technique de dosage.

Selon l'invention, les nanoparticules peuvent être en suspension dans une solution saline ou un tampon biologique. Des essais ont notamment été réalisés en tampon PBS (Phosphate Buffered Saline), en tampon TRIS (trishydroxyméthylaminomethane), ou dans une solution saline comprenant 90g/l de NaCl, 0.12g/l de Na₂HPO₄, et 0.6g/l de KH₂PO₄.

Selon une caractéristique particulière de l'invention, la suspension comprenant les nanoparticules de pseudo-boehmite et de polyacrylate comprend également un tensioactif, notamment un tensioactif neutre tel que le Brij^{™} 58, le Pluronic^{™}123 ou le Tween^{™}60, ou un tensioactif anionique tel que le SDS (dodecyl sulfate de sodium), ou encore un polymère tel que le PEG (polyéthylène glycol). Ainsi, on peut augmenter la stabilisation des nanoparticules formées par effet stérique ou par répulsion électrostatique.

Selon l'invention, la composition vaccinale comprend au moins un antigène.

Aux fins de mise au point de la présente invention, on a utilisé la protéine tétanique purifiée comme antigène-modèle. Des essais ont ensuite été faits avec d'autres antigènes, notamment des antigènes de la grippe, l'antigène LSA3 de malaria, ou encore des antigènes recombinants de ETEC (Enterotoxigenic *Escherichia coli).* Cependant la composition vaccinale selon l'invention peut comprendre n'importe quel antigène pouvant être utilisé dans un vaccin, qu'il s'agisse d'un germe entier, d'un antigène sous-unitaire, naturel, recombinant, hybride,... peu important sa nature ; l'antigène peut en effet être un peptide, une protéine, une glycoprotéine, un polysaccharide, un glycolipide, un lipopeptide, une VLP (virus-like particle)...etc.

Ces antigènes sont des antigènes utilisés ou susceptibles d'être utilisés pour le traitement ou la prévention de diverses maladies susceptibles d'atteindre le monde animal et en particulier les être humains, notamment : diphtérie, tétanos, polio, rage, coqueluche, hépatites A, B, C, fièvre jaune, fièvre typhoïde, varicelle, rougeole, oreillons, rubéole, encéphalite japonaise, grippe, méningites, choléra, infections à : Rotavirus, Norovirus, Rhinovirus, Respiratory Syncytial Virus, Herpes Simplex Virus, Papilloma Virus, Cytomegalovirus, West Nile Virus, Dengue Virus, Chykungunya Virus,HIV (SIDA), les affections bactériennes provoquées par : des streptocoques, Chlamydia trachomatis et pneumoniae, Neisseria gonorrheae et meningitidis, Moraxella catarrhalis, Staphylococcus aureus ou Haemophilus influenza type B, les listerioses, les shigelloses, les salmonelloses, la tuberculose, la maladie de Lyme, le cancer, les affections parasitaires telles que la malaria, les leshmanioses ...etc.

La composition pharmaceutique selon l'invention peut être une composition destinée à l'immunisation contre un seul pathogène ou cancer, c'est-à-dire qu'elle comprend un ou plusieurs antigènes d'un seul pathogène ou cancer, ou bien être une composition destinée à l'immunisation contre plusieurs pathogènes ou cancers (on parle alors de combinaison vaccinale). La composition selon l'invention peut également comprendre plusieurs antigènes spécifiques d'une seule maladie, mais appartenant à différentes catégories de l'agent de cette maladie (plusieurs souches ou sérotypes, ou clades, suivant la nature de l'agent). Il peut également s'agir d'une composition vaccinale comprenant des allergènes, destinée notamment à une désensibilisation dans le cadre du traitement des allergies.

La composition vaccinale selon l'invention peut être administrée par toutes les voies habituellement utilisées pour l'administration des vaccins ; cependant, elle est d'un intérêt particulier pour la voie intra-dermique. En effet, la voie intra-dermique, si elle semble très efficace pour induire de bonnes réactions immunitaires, a pour inconvénient de conduire parfois à des réactions de réactogénicité locale, qui peuvent freiner son utilisation. Grâce aux nanoparticules selon l'invention, il a été possible de réaliser des immunisations par voie intradermique en n'ayant que très peu ou pas du tout de réaction de réactogénicité, et notamment pas d'effet tatouage ainsi que cela avait pu être le cas avec les suspensions d'aluminium de l'art antérieur.

Selon l'invention, on prépare la composition vaccinale par simple mélange d'une suspension comprenant les nanoparticules de pseudo-boehmite et de polyacrylate et une suspension d'antigènes. Cette opération peut se faire par ajout des antigènes dans une suspension colloïdale comprenant les nanoparticules ou par ajout des nanoparticules dans une suspension comprenant déjà les antigènes. D'autre part, dans le cas où on souhaite disposer de compositions vaccinales comprenant plusieurs types d'antigènes, il peut être préféré d'effectuer en priorité l'adsorption de certains antigènes par rapport à d'autres. Notamment, dans le cas où la composition vaccinale comprend un mélange d'antigènes dont certains, pour des raisons de stabilité ou d'immunogénicité, ne doivent pas être adsorbés, il peut être préférable de procéder de la manière suivante : on sature tout d'abord les particules à base d'aluminium avec les antigènes nécessitant une adsorption ou avec des ions ou excipients présents dans la substance tampon avant d'introduire les antigènes qui ne doivent pas être adsorbés.
Lors de la préparation des nanoparticules selon l'invention, il est possible qu'il y ait du polymère en excès. Pour éliminer cet éventuel excès de polymère, il est possible de procéder à une étape supplémentaire, soit directement après la phase de préparation des nanoparticules, soit après la phase de mélange avec l'antigène. Cette phase d'élimination peut être réalisée par dialyse ou diafiltration contre de l'eau déminéralisée (Milli Q, Millipore), une solution saline ou un tampon biologique, au travers d'une membrane de porosité adéquate (de 30 à 100 kDa suivant la taille des nanoparticules).
Les exemples qui suivent illustrent des modes de réalisation de l'invention.

### Exemple 1 : Synthèse d'une suspension colloïdale de nanoparticules de pseudo-boehmite

On a utilisé du polyacrylate de sodium (NaPa) de masse moléculaire 2100 fourni par Fluka et du chlorure d'aluminium hexahydraté (AlCl₃. 6H₂O) fourni par Avocado. On a dissous 9g de NaPa dans 75 ml d'une solution aqueuse de AlCl₃ 0.1M. Le mélange résultant a été agité vigoureusement à température ambiante pendant 24h. Le pH du mélange était alors compris entre 5.5 et 5.9. On a ensuite ajouté goutte à goutte de la soude NaOH 5M jusqu'à obtention d'un pH de 10.5 puis on a encore agité pendant 10 min. Lorsque l'augmentation de pH a atteint 9.0-9.5, la solution est devenue légèrement turbide, ce qui correspond à la précipitation des nanoparticules de pseudo-boehmite. Pour obtenir les nanoparticules finales, la suspension obtenue à pH 10.5 a été transférée dans un autoclave et chauffée à 160°C sous faible agitation (15 trs/min) pendant 3 heures. Ensuite, la suspension colloïdale a été lavée par dialyse contre 51 d'eau distillée grâce à un dispositif Sartorius Slice 200 Benchtop équipé d'une membrane en polyéther polysulfone de 30 kDa. La suspension colloïdale résultante a été stockée dans des flacons en polypropylène. Les mesures de taille effectuées ont montré que l'on était en présence d'une population unique de nanoparticules dont la taille allait de 15 à 40 nm (distribution en nombre), avec un index de polydispersité de 0.19 (analyse en intensité). Un échantillon de matériel solide a été obtenu par centrifugation à 25000 trs/min pendant 1 heure et séché à 80°C pendant toute une nuit, à des fins d'analyse physico-chimique.

L'échantillon a été analysé par diffraction de rayons X au moyen d'un diffractomètre STOE STADI-P utilisant la raie Kα1 du cuivre (λ=1.5406Å). Le rayonnement est parfaitement monochromatique grâce à la présence d'un monochromateur avant constitué d'un cristal de germanium. Pour cette analyse, les échantillons, préalablement broyés, sont placés dans des tubes de Lindemann (0.3mm de diamètre). Le tube capillaire, placé sur une tête goniométrique, est mis en rotation et le diffractogramme est enregistré en mode Debye-Scherrer à l'aide d'un détecteur linéaire court de type PSD (Position Sensitive Detector) pouvant couvrir une plage angulaire de 11° (2θ).
L'analyse de diffraction aux rayons X de l'échantillon séché a donné un résultat caractéristique de pseudo-boehmite.
Les nanoparticules obtenues ont pu être filtrées stérilement sur membrane 0.2µm en PVDF de Millipore, avec une perte minimale en matériel (3.1 %) déterminée par absorption atomique.

### Exemple 2 : Synthèse de nanoparticules de pseudo-boehmite et de polyacrylate à partir d'une solution de Polyacrylate de Sodium de masse molaire 60 000.

On a utilisé du polyacrylate de sodium (NaPa) de masse moléculaire 60000 fourni par Polysciences sous forme d'une solution aqueuse à 35% (w/w) et du chlorure d'aluminium hexahydraté (AlCl₃. 6H₂O) fourni par Fluka.

Afin d'obtenir 36g de polymère on a prélevé 102.86g de solution polymérique qui contenait 66.86g d'eau.

Dans un bécher, on a introduit 7.2429g d'AlCl₃ auxquels on a ajouté 225.89g d'eau.

On a ajouté cette solution d'aluminium à la solution polymérique, afin d'obtenir 300 ml d'une solution 0.1M en AlCl₃.

Le mélange obtenu était blanc.

On a agité ce mélange fortement pendant 24 heures.

Après 24 heures, le pH du mélange était de 6.1. On a ensuite ajouté goutte à goutte de la soude NaOH 5M jusqu'à un pH de 10.2.

Le mélange a été homogénéisé sous agitation pendant 1 heure, puis placé sous agitation (1400 tours/min) dans un autoclave et chauffé à 160°C pendant 3 heures.

La solution obtenue était légèrement opalescente, et a été analysée pour montrer qu'elle était constituée de nanoparticules présentant par diffraction de rayons X des pics caractéristiques de la pseudo-boehmite, et une taille moyenne autour de 100 nm, compatible avec une filtration stérilisante.

La solution a pu être conservée pendant plus d'1 an en maintenant l'intégrité des nanoparticules qui ne se sont pas réagrégées.

### Exemple 3 : Préparation d'une composition vaccinale comprenant des nanoparticules d'hydroxyde d'aluminium et de la protéine tétanique purifiée, et test de filtration.

On a préparé une composition vaccinale en ajoutant 10 µl d'une préparation de protéines tétaniques purifiées dosée à une concentration de 1200 unités de floculation/ml de solution saline à 4.5 ml d'une suspension de nanoparticules de pseudo-boehmite préparée selon l'exemple 1.

Le mélange a été agité modérément puis passé au travers d'une membrane en PVDF (fournie par Millipore) montée sur une seringue plastique de 5ml.

Une mesure de la taille des nanoparticules a été réalisée à la fois avant, et après l'addition de protéine tétanique sur les nanoparticules ; on a obtenu le même profil de taille, que ce soit en présence ou en absence de protéines, ce qui démontre que la protéine tétanique ne conduit pas à l'agrégation des nanoparticules.

De même, on a déterminé que la filtration conduisait à une perte en aluminium de 3%, ce qui est tout à fait acceptable d'un point de vue industriel.

On peut donc en conclure que les compositions vaccinales selon l'invention peuvent être filtrées sans trop de perte de matière sur un filtre stérilisant de 0.22 µm.

### Exemple 4 : Test de réactogénicité sur souris d'une composition selon l'invention comprenant des nanoparticules et des antigènes de la grippe, administrée par voie intradermique.

On a utilisé comme modèle-animal pour ce test, des souris BALB/c femelles afin d'évaluer la réactogénicité locale de compositions selon l'invention, comparativement aux suspensions d'aluminium classiques.

Dans ce test, l'antigène est constitué par un vaccin grippe trivalent appelé Flu ID stock comprenant les souches fragmentées inactivées A/Solomon/3/2006 (H1N1), A/Wisconsin/67/2005 (H3N2), et B/Malaysia/2506/2004 à raison de 150 µg/ml de HA par souche.

4 groupes de 10 souris ont reçu à 3 semaines d'intervalle, par voie intradermique, dans la face interne de l'oreille, une dose de 30 µl sub-optimale (soit 0.3µg de HA/ souche) de vaccin en présence ou non d'un adjuvant.

Les compositions administrées ont été préparées de la manière suivante :
- Groupe A : Flu ID seul ; on a dilué 54 µl de Flu ID stock dans 756 µl de tampon PBS.
- Groupe B : Flu ID + AlOOH ; on a ajouté successivement :
   ∘ 54 µl de Flu ID stock
   ∘ 604 µl de tampon PBS
   ∘ 152 µl d'une suspension commerciale d'AlOOH à 8.01mg/ml d'aluminium (Alhydrogel®)
Le mélange a été agité modérément pendant 2 heures.
- Groupe C : Flu ID + AlPO₄ ; on a ajouté successivement :
   ∘ 54 µl de Flu ID stock
   ∘ 463 µl de tampon PBS
   ∘ 293 µl d'une suspension commerciale d'AlPO₄ à 4.15mg/ml d'aluminium (AdjuPhos®)
Le mélange a été agité modérément pendant 2 heures.
- Groupe D : Flu ID + nanoAlOOH ;on a ajouté successivement :
   ∘ 54 µl de Flu ID stock
   ∘ 5 µl de H₂O
   ∘ 76 µl de tampon PBS concentré 10 fois
   ∘ 675 µl d'une suspension de nanoparticules de pseudo-boehmite, préparée de la manière décrite à l'exemple 1 et comprenant 1.8mg d'Al/ml
Le mélange a été agité modérément pendant 2 heures à température ambiante.

Les souris ont été surveillées chaque jour, et on a noté sur une échelle non-officielle les oedèmes, les érythèmes et les lésions apparaissant à l'oreille, pendant 2 semaines après chaque injection.
Quelle que soit la formulation testée, aucun érythème significatif n'a été observé. De même, on n'a signalé aucun oedème.
Par contre, on a noté au point d'injection des nodules blancs/rougeâtres apparaissant chez les souris ayant reçu les compositions comprenant de l'aluminium classique, que ce soit avec l'hydroxyde d'aluminium, ou avec le phosphate d'aluminium. Mais, de manière surprenante et très intéressante, aucun nodule n'est visible sur les souris ayant reçu une composition selon l'invention.
Les résultats relatifs aux nombres de souris présentant des nodules après administration des adjuvants comprenant l'aluminium de l'art antérieur, sont illustrés sur le tableau 1 dessous :

**Tableau 1**

| **Après la 1^{ère} injection (J0)** | | | | | | | | | | | |
|---|---|---|---|---|---|---|---|---|---|---|---|
| **Adjuvant** | **J1** | **J2** | **J3** | **J4** | **J7** | **J8** | **J9** | **J10** | **J11** | **J15** | |
| **AlOOH** | 0/10 | 0/10 | 0/10 | 0/10 | 6/10 | 10/10 | 10/10 | 10/10 | 10/10 | 8/10 | |
| **AlPO₄** | 0/10 | 1/10 | 2/10 | 3/10 | 6/10 | 9/10 | 9/10 | 9/10 | 10/10 | 9/10 | |

| **Après le rappel (D21)** | | | | | | | | | | | |
|---|---|---|---|---|---|---|---|---|---|---|---|
| **Adjuvant** | **J22** | **J23** | **J24** | **J25** | **J28** | **J29** | **J30** | **J31** | **J32** | **J35** | **J39** |
| **AlOOH** | 5/10 | 9/10 | 10/10 | 9/10 | 10/10 | 10/10 | 10/10 | 10/10 | 10/10 | 8/10 | 9/10 |
| **AlPO₄** | 9/10 | 9/10 | 8/10 | 7/10 | 9/10 | 10/10 | 10/10 | 10/10 | 10/10 | 10/10 | 10/10 |

Il est particulièrement intéressant de constater que, malgré le fait que les quantités d'aluminium soit les mêmes (45µg) dans tous les groupes, le fait de le formuler en nanoparticules selon l'invention, rend cet aluminium bien mieux toléré.

### Exemple 5 : Test d'immunogénicité sur souris d'une composition selon l'invention comprenant des nanoparticules et des antigènes de la grippe, administrée par voie intradermique.

Dans ce test, on a évalué l'immunogénicité des compositions selon l'invention, comparativement à des compositions comprenant de l'aluminium classique, que ce soit AlOOH ou AlPO₄, mais également vis-à-vis de composition ne comprenant pas d'aluminium mais uniquement du polymère.

Dans ce test, comme dans l'exemple précédent, l'antigène était constitué par un vaccin grippe trivalent appelé Flu ID stock comprenant les souches fragmentées inactivées A/Solomon/3/2006 (H1N1), A/Wisconsin/67/2005 (H3N2), et B/Malaysia/2506/2004 à raison de 150 µg/ml de HA par souche. Le lot de vaccin Flu ID stock utilisé pour ce test était le même que celui utilisé dans l'exemple précédent.

Le test a été réalisé sur des souris BALB/c femelles réparties en 5 groupes de 9. Chaque souris a reçu, à 3 semaines d'intervalle, par voie intradermique, dans la face interne de l'oreille, une dose de 30 µl sub-optimale (soit 0.3µg de HA/ souche) d'une composition telle que décrite ci-après, en fonction de son groupe d'appartenance.

Les compositions administrées ont été préparées de la manière suivante :
- Groupe A : Flu ID seul ; on a dilué 54 µl de Flu ID stock dans 756 µl de tampon PBS.
- Groupe B : Flu ID + AlPO₄ ; on a ajouté successivement :
   ∘ 54 µl de Flu ID stock
   ∘ 463 µl de tampon PBS
   ∘ 293 µl d'une suspension commerciale d'AlPO₄ à 4.15mg/ml d'aluminium (AdjuPhos®)
Le mélange a été vortexé pendant 10 secondes.
- Groupe C : Flu ID + AlOOH ; on a ajouté successivement :
   ∘ 54 µl de Flu ID stock
   ∘ 595 µl de tampon PBS
   ∘ 161 µl d'une suspension commerciale d'AlOOH à 7.53mg/ml d'aluminium (Alhydrogel®)
   Le mélange a été vortexé pendant 10 secondes.
- Groupe D : Flu ID + nanoparticules de pseudo-boehmite ; on a ajouté successivement :
   ∘ 54µl de Flu ID stock,
   ∘ 5 µl de H₂O
   ∘ 76 µl de tampon PBS concentré 10 fois,
   ∘ 675 µl de suspension de nanoparticules de pseudo-boehmite, préparée de la manière décrite à l'exemple 1 et comprenant 1.8mg d'Al/ml.
   Le mélange a été vortexé pendant 10 secondes.
- Groupe E : Flu ID + polymère ; on a ajouté successivement :
   ∘ 54µl de Flu ID stock,
   ∘ 5 µl de H₂O
   ∘ 76 µl de tampon PBS concentré 10 fois,
   ∘ 675 µl d'une solution à 17mg/ml de PaNa (polyacrylate de sodium) de masse molaire 2100 fourni par Fluka
   Le mélange a été vortexé pendant 10 secondes.

On a surveillé les souris durant tout le test.
Des échantillons sanguins de chaque souris ont été prélevés à J42, soit 3 semaines après la 2nde injection, par section de la carotide. Les échantillons ont été traités afin d'isoler le sérum pour effectuer les tests de réponse humorale.
On a en outre prélevé les rates de 6 souris par groupe afin d'effectuer les tests de réponse cellulaire.
Les tests qui ont été réalisés sont des dosages ELISA, des tests d'inhibition de l'hémagglutination, ainsi que des ELISPOT.
Les dosages ELISA ont été réalisés de façon classique, afin de déterminer les quantités d'IgG1 et d' IgG2a sériques spécifiques de la souche A/H1N1. Le seuil de détection des anticorps est de 20 (1.3log10) unités ELISA. Tous les titres sont exprimés en log10 d'unités ELISA. Pour chaque groupe d'animaux, on a calculé la moyenne géométrique ainsi que l'intervalle de confiance 95% correspondant.
Le test d'inhibition de l'hémagglutination permet d'apprécier les anticorps fonctionnels présents dans le sérum des animaux immunisés. Il mesure la capacité des anticorps induits à inhiber l'hémagglutination de globules rouges de poulet par le virus grippal étudié. Le titre d'inhibition de l'hémagglutination ou HI (Haemagglutination Inhibition) est l'inverse de la dernière dilution pour laquelle on n'observe pas d'hémagglutination. Pour chaque groupe d'animaux, on a calculé la moyenne géométrique ainsi que l'intervalle de confiance à 95% correspondant. Ceci vis-à-vis de chacune des 3 souches présentes dans la composition administrée.

Les dosages ELISPOT sont réalisés à partir des cellules spléniques fraichement isolées, mises à incuber la nuit et restimulées avec un mélange des 3 souches de la composition vaccinale ou par un peptide 9-mer correspondant à un épitope de classe I (épitope CD8) de la protéine NP. Les réponses cellulaires sont exprimées en nombres de cellules sécrétant de l'IL-5 ou de l'IFN-γ spécifiques de la grippe, pour 10⁶ splénocytes.
Lors de la restimulation avec le peptide spécifique de la grippe, on n'a pas détecté, par ELISPOT de cellules T CD8+ sécrétant de l'IL-5 ou de l'IFN-γ ; ceci, quel que soit le groupe de souris considéré.

En ce qui concerne la restimulation in-vitro par les antigènes de la grippe, les résultats obtenus sont représentés dans le tableau 2 ci-après, où les intervalles de confiance 95% sont indiqués entre crochets :

**Tableau 2 :**

| Groupe considéré | Nbre de cellules sécrétrices d'IL-5 /10⁶ splénocytes | Nbre de cellules sécrétrices d'IFN-γ/10⁶ splénocytes |
|---|---|---|
| A : Flu ID seul | 24 [12 - 48] | 2 [0 - 17] |
| B : Flu ID + AlPO₄ | 17 [6 - 45] | 2 [0 - 32] |
| C : Flu ID + AlOOH | 46 [19 - 115] | 13 [5 - 38] |
| D : Flu ID + nanoparticules | 197 [79-491] | 79 [30-205] |
| E : Flu ID + polyacrylate | 80 [42-151] | 8 [1- 81] |

Ces résultats montrent que, de façon surprenante, grâce aux nanoparticules selon l'invention, il est possible d'obtenir une réponse cellulaire, notamment une stimulation des cellules sécrétrices d'IL-5 ainsi que des cellules sécrétrices d'IFN-γ.

D'un point de vue statistique, selon un modèle mixte avec ajustement de Dunnett, les nanoparticules sont considérées avoir augmenté significativement le nombre de cellules sécrétrices d'IL-5 et d'IFN-γ, de 8.2 fois (p<0.001) et de 39.5 fois (p=0.002), respectivement, ce qui n'est pas le cas des adjuvants à base d'aluminium de l'art antérieur.

On remarque en outre que le polyacrylate seul n'a augmenté la sécrétion d'IL5 que de 3.3 fois (p=0.027), à la limite de la significativité.

Les résultats relatifs aux tests de réponse humorale sont repris dans le tableau 3 ci-après où, à chaque fois, les intervalles de confiance 95% sont mis entre crochets.

**Tableau 3 :**

| Groupe considéré | HI contre H1N1 | HI contre H3N2 | HI contre B | IgG1 contre H1N1 (log10) | IgG2a contre H1N1 (log10) |
|---|---|---|---|---|---|
| A : Flu ID seul | 101 | 127 | 17 | 4.8 | 4.3 |
| | [52 - 194] | [66 - 244] | [6 - 46] | [4.4 - 5.2] | [3.9 - 4.6] |
| B : Flu ID + AlPO₄ | 1881 | 1097 | 593 | 6.4 | 4.4 |
| | [1096 - 3229] | [654 - 1842] | [301 -1165] | [6.2 - 6.7] | [3.9 - 4.9] |
| C : Flu ID + AlOOH | 1185 | 1185 | 640 | 6.4 | 4.1 |
| | [637 - 2207] | [603 - 2331] | [353 -1161] | [6.1 - 6.6] | [3.4 - 4.8] |
| D:FIuID+ *nanoparticules* | 1185 | 1613 | 508 | 6.1 | 5.1 |
| | [676 - 2078] | [1017 - 2558] | [228 -1130] | [5.9 - 6.4] | [4.7 - 5.4] |
| C : Flu ID + polyacrylate | 93 | 148 | 13 | 5.0 | 4.1 |
| | [49 - 177] | [90 - 243] | [4 - 38] | [4.8 - 5.3] | [3.7 - 4.5] |

D'un point de vue statistique, l'augmentation des titres HI et des titres en IgG1 obtenus avec les nanoparticules a été considérée significative vis-à-vis des titres obtenus avec le vaccin seul selon un modèle mixte avec ajustement de Dunnet (11.7 fois pour titre HI anti-H1N1 avec p< 0.001 ; 12.7 fois pour titre HI anti-H3N2 avec p<0.001 ; 29.9 fois pour titre anti-B avec p< 0.001 ; 20.0 fois pour titre IgG1 anti-H1N1 avec p< 0.001 et 6.3 fois pour titre IgG2a anti-H1N1 avec p=0.008).

Ces résultats montrent la capacité des nanoparticules selon l'invention à induire une réponse humorale après 2 immunisations par voie intradermique. On peut remarquer que le polymère seul n'a pas ce pouvoir adjuvant.

D'autre part, seules les nanoparticules selon l'invention conduisent à une augmentation de la réponse en IgG2a, qui signifie que le profil de la réponse immunitaire est plus orientée vers une réponse de type TH1 que les autres adjuvants à base d'aluminium.

## Revendications

1. Composition vaccinale comprenant au moins un antigène et un adjuvant, **caractérisée en ce que** l'adjuvant comprend des nanoparticules comprenant de la pseudo-boehmite et du polyacrylate, lesdites nanoparticules ayant une taille permettant leur passage à travers un filtre stérilisant dont le seuil de coupure est de 220nm.

2. Composition vaccinale selon la revendication 1, **caractérisée en ce que** le noyau de la nanoparticule est constitué essentiellement de pseudo-boehmite, et **en ce que** le polyacrylate est situé essentiellement en surface des nanoparticules.

3. Composition vaccinale selon une des revendications précédentes, **caractérisée en ce que** la pseudo-boehmite représente au moins 90% de la masse des nanoparticules.

4. Composition vaccinale selon une des revendications précédentes, **caractérisée en ce que** la taille des nanoparticules est inférieure à 300 nm, avantageusement inférieure à 220 nm.

5. Composition vaccinale selon une des revendications précédentes, **caractérisée en ce qu'**elle comprend au moins un antigène de la grippe.

6. Composition vaccinale selon une des revendications précédentes, **caractérisée en ce qu'**elle comprend au moins une protéine tétanique.

7. Nanoparticules comprenant de la pseudo-boehmite et du polyacrylate, lesdites nanoparticules ayant une taille permettant leur passage à travers un filtre stérilisant dont le seuil de coupure est de 220nm, pour leur utilisation dans une composition vaccinale comprenant au moins un antigène.

8. Nanoparticules selon la revendication précédente, **caractérisées en ce que** la composition vaccinale est destinée à être administrée par voie intradermique.

9. Nanoparticules selon une des revendications 7 ou 8, **caractérisées en ce que** la composition vaccinale comprend au moins un antigène de la grippe.

10. Nanoparticules selon une des revendications 7 à 9, **caractérisées en ce que** lesdites nanoparticules permettent d'augmenter la réponse immunitaire induite lors de l'administration de ladite composition vaccinale.

11. Procédé de préparation d'une composition vaccinale selon une des revendications 1 à 6, selon lequel :
∘ on prépare des nanoparticules comprenant de la pseudo-boehmite et du polyacrylate, lesdites nanoparticules ayant une taille permettant leur passage à travers un filtre stérilisant dont le seuil de coupure est de 220nm,
∘ on filtre lesdites nanoparticules au moyen d'un filtre stérilisant,
∘ on ajoute auxdites nanoparticules au moins un antigène vaccinal.

12. Procédé de préparation selon la revendication précédente, **caractérisé en ce qu'**on réalise une filtration supplémentaire après addition dudit antigène vaccinal.

## Patentansprüche

1. Impfstoffzusammensetzung, umfassend mindestens ein Antigen und ein Adjuvans, **dadurch gekennzeichnet, dass** das Adjuvans Nanopartikel umfasst, die Pseudoböhmit und Polyacrylat umfassen, wobei die Nanopartikel eine Größe aufweisen, die ihnen den Durchtritt durch ein Sterilisationsfilter mit einer Ausschlussgröße von 220 nm gestattet.

2. Impfstoffzusammensetzung nach Anspruch 1, **dadurch gekennzeichnet, dass** der Kern des Nanopartikels im Wesentlichen aus Pseudoböhmit besteht, und dadurch, dass das Polyacrylat sich im Wesentlichen an der Oberfläche der Nanopartikel befindet.

3. Impfstoffzusammensetzung nach einem der vorhergehenden Ansprüche, **dadurch gekennzeichnet, dass** das Pseudoböhmit mindestens 90% der Masse der Nanopartikel ausmacht.

4. Impfstoffzusammensetzung nach einem der vorhergehenden Ansprüche, **dadurch gekennzeichnet, dass** die Größe der Nanopartikel weniger als 300 nm, vorzugsweise weniger als 220 nm beträgt.

5. Impfstoffzusammensetzung nach einem der vorhergehenden Ansprüche, **dadurch gekennzeichnet, dass** sie mindestens ein Influenza-Antigen umfasst.

6. Impfstoffzusammensetzung nach einem der vorhergehenden Ansprüche, **dadurch gekennzeichnet, dass** sie mindestens ein Tetanusprotein umfasst.

7. Nanopartikel, umfassend Pseudoböhmit und Polyacrylat, wobei die Nanopartikel eine Größe aufweisen, die ihnen den Durchtritt durch ein Sterilisationsfilter mit einer Ausschlussgröße von 220 nm gestattet, für die Verwendung in einer Impfstoffzusammensetzung, die mindestens ein Antigen umfasst.

8. Nanopartikel nach dem vorhergehenden Anspruch, **dadurch gekennzeichnet, dass** die Impfstoffzusammensetzung für die intradermale Verabreichung bestimmt ist.

9. Nanopartikel nach einem der Ansprüche 7 oder 8, **dadurch gekennzeichnet, dass** die Impfstoffzusammensetzung mindestens ein Influenza-Antigen umfasst.

10. Nanopartikel nach einem der Ansprüche 7 bis 9, **dadurch gekennzeichnet, dass** es die Nanopartikel gestatten, die bei der Verabreichung der Impfstoffzusammensetzung induzierte Immunantwort zu steigern.

11. Verfahren zur Herstellung einer Impfstoffzusammensetzung nach einem der Ansprüche 1 bis 6, bei dem man:
∘ Nanopartikel herstellt, die Pseudoböhmit und Polyacrylat umfassen, wobei die Nanopartikel eine Größe aufweisen, die ihnen den Durchtritt durch ein Sterilisationsfilter mit einer Ausschlussgröße von 220 nm gestattet,
∘ die Nanopartikel mithilfe eines Sterilisationsfilters filtriert,
∘ zu den Nanopartikeln mindestens ein Impfantigen hinzugibt.

12. Herstellungsverfahren nach dem vorhergehenden Anspruch, **dadurch gekennzeichnet, dass** man eine zusätzliche Filtration nach der Zugabe des Impfantigens durchführt.

## Claims

1. Vaccine composition comprising at least one antigen and an adjuvant, **characterized in that** the adjuvant comprises nanoparticles comprising pseudo-boehmite and polyacrylate, said nanoparticles having a size which allows them to pass through a sterilizing filter with a cut-off threshold of 220 nm.

2. Vaccine composition according to Claim 1, **characterized in that** the core of the nanoparticle consists essentially of pseudo-boehmite, and **in that** the polyacrylate is essentially located at the surface of the nanoparticles.

3. Vaccine composition according to either of the preceding claims, **characterized in that** pseudo-boehmite represents at least 90% of the mass of the nanoparticles.

4. Vaccine composition according to one of the preceding claims, **characterized in that** the size of the nanoparticles is less than 300 nm and advantageously less than 220 nm.

5. Vaccine composition according to one of the preceding claims, **characterized in that** it comprises at least one influenza antigen.

6. Vaccine composition according to one of the preceding claims, **characterized in that** it comprises at least one tetanus protein.

7. Nanoparticles comprising pseudo-boehmite and polyacrylate, said nanoparticles having a size which allows them to pass through a sterilizing filter with a cut-off threshold of 220 nm, for their use in a vaccine composition comprising at least one antigen.

8. Nanoparticles according to the preceding claim, **characterized in that** the vaccine composition is intended to be administered intradermally.

9. Nanoparticles according to either of Claims 7 and 8, **characterized in that** the vaccine composition comprises at least one influenza antigen.

10. Nanoparticles according to one of Claims 7 to 9, **characterized in that** said nanoparticles make it possible to increase the immune response induced during the administration of said vaccine composition.

11. Process for preparing a vaccine composition according to one of Claims 1 to 6, according to which:
∘ nanoparticles comprising pseudo-boehmite and polyacrylate are prepared, said nanoparticles having a size which allows them to pass through a sterilizing filter with a cut-off threshold of 220 nm.
∘ said nanoparticles are filtered by means of a sterilizing filter,
∘ at least one vaccine antigen is added to said nanoparticles.

12. Preparation process according to the preceding claim, **characterized in that** after addition of said vaccine antigen an additional filtration is performed.
